# EUROPEAN PATENT SPECIFICATION

(11) **EP 2 280 756 B1**
(45) Date of publication and mention of the grant of the patent: **23.07.2014**
(21) Application number: 08767037.8
(22) Date of filing: 08.05.2008
(51) Int. Cl.: A61N 1/05, H01B 7/02

(54) **AN IMPLANTABLE MEDICAL LEAD AND A METHOD OF MANUFACTURING THEREOF**
IMPLANTIERBARE MEDIZINISCHE LEITUNG UND VERFAHREN ZU IHRER HERSTELLUNG
FIL MÉDICAL IMPLANTABLE ET SON PROCÉDÉ DE FABRICATION

(30) Priority: 29.04.2008 WO PCT/SE2008/000299
(43) Date of publication of application: 09.02.2011
(73) Proprietor: St. Jude Medical AB, 175 84 Järfälla (SE)
(72) Inventor: DOWLING, Kenneth, SE-197 91 Bro (SE)
(74) Representative: Sjölander, Henrik
(86) International application number: PCT/SE2008/000313
(87) International publication number: WO 2009/134171

(56) References cited:
- EP-A2- 1 331 016
- US-A- 5 316 706
- US-A- 5 316 706
- US-A- 5 484 565
- US-A- 5 695 482
- US-A- 5 733 496
- US-A- 5 871 468
- US-A1- 2002 156 383
- US-A1- 2003 139 760
- US-A1- 2005 228 161
- US-A1- 2005 228 161
- US-A1- 2005 277 878
- US-A1- 2006 282 144
- US-A1- 2007 106 336
- US-A1- 2007 106 336
- US-A1- 2007 184 197
- US-A1- 2008 046 059
- US-B1- 6 188 931
- US-B2- 7 091 297

## Description

### Field of the invention

The present invention generally relates to the field of implantable medical devices. More specifically, the present invention relates to an implantable medical lead for implantation in a patient, the lead having a distal end adapted to be located within or at a heart of the patient and a proximal end connectable to an implantable medical device.

### Background of the invention

Within the field of implantable medical device, such as heart stimulators or pacemakers, implantable leads are used for conveying electrical stimuli from the device to a distal portion of the lead, e.g. to the myocardium of a human heart, for instance the endocardium and to transfer signals, for example, signal representative of electrical activity of the heart to the device. The requirements of cardiac leads with respect to material properties or characteristics are contradictory which may be difficult to combine. For example, a distal portion of the cardiac lead needs to be flexible yet having a satisfactory degree of stiffness such that the portion easily can adapt to and follow the curvature of the implantation path within the vessels and to avoid perforation or tearing of vessel walls and tissue walls, such as a heart wall. Conversely, the remaining portion and in particular the proximal portion or end is relatively stiff or rigid to ease insertion of the lead during implantation procedure, i.e. a manipulation of the proximal end allows the distal end to be operated. Further, the proximal portion which is connected to the device should also have a wear resistible surface to cope with the wear due to friction between the proximal portion and the can of the device. Thus, because of the compromise between the different material properties required by the material used in a cardiac lead, this is a complex issue.

As indicated above, a problem is abrasion or wear of the portion of the lead that is in contact with the medical device when implanted. More specifically, during a implantation procedure extra or additional lead wire is provided at the site where the medical device is implanted. This is a security measure for the purpose of compensating body movements which otherwise could cause stretching of the lead. Since the medical device, which conventionally is implanted in a subcutaneous pocket, is also more or less fixated, such stretching could, in absence of excess lead wire, cause a stressed distal end. As a result, the portion of the distal end, including e.g. a helix, that is secured to a target tissue, e.g. a heart wall, could cause damages to the tissue. Thus, a coil of excess lead wire is conventionally implanted together with medical device to avoid that situation, which achieves a resilient effect between the two fixation points of the distal end and proximal end of the lead.

However, when implanted the medical lead abuts against the surface of the medical device since they are located close to each other and friction between the can of the device and the lead portion abutting the can due to, for example, body movements may cause wear on the lead surface. Thereby, the lead surface which is subjected to wear or abrasion needs to be provided with an inherent resistance against such wearing and tearing.

In practice, a material is often selected as a compromise between wear resistant and flexibility properties, being optimized for neither.

A summing-up of the presented problems result in an implantable medical lead having a distal end portion which is flexible and a proximal portion sufficiently rigid to manoeuvre the distal end and wherein at least the proximal end portion is abrasion resistant. In the prior art, the medical lead may be constructed by individual portions, a distal portion and a proximal portion which are assembled by means of a intermediate joint or seem. These individual parts or components may made of different material and individually treated to obtain a desirable mechanical property suitable for its purpose. However, such a solution only solves part of the complex problem, but more important this solution increases significantly the complexity of joining these components and the difficulty of assembly and manufacturing thereof.

One way of addressing part of the problem presented above is done by Sagae et al. in prior art document US 5 171 383. Here a catheter guide wire is disclosed wherein a core member is made of an elastic alloy. This elastic alloy is subjected to a heat treatment process along its longitudinal direction such that the rigidity of the proximal end portion becomes comparatively high and the flexibility of the distal end is increased. This differential heat treatment provides a catheter guide wire having a flexible distal end to avoid buckling deformations and tissue wall perforations, and a rigid proximal end to achieve a good torque transmitting performance to the distal end portion. However, this solution does not solve the problem of wear of the surface of the lead.

In US 4 963 306 by Weldon, another prior art technique is disclosed which presents a method for making a fuseless soft tip angiographic catheter. A fuseless polymeric tube having a body portion and a tip portion is provided, wherein the body portion is heat treated while the tip portion is maintained at a temperature lower than the heat treatment temperature. Thereby, a polymeric tube having a tip portion and a body portion with different physical properties. Thus, the soft tip is flexible such that the catheter may is able to reach distant vessels without damaging or tearing the lining of the blood vessels. However, this is a catheter device for an insertion procedure and not intended for implantation. Also, this prior art does not address the problem related to the wear of the surface of the lead.

Consequently, there is a need within the art of implantable medical leads that enables a durable and reliable implantable medical lead in combination with an accurate and easy implantation procedure thereof.

US 5,316,706 discloses a catheter formed from a single extrusion of cold crystallisable material in its amorphous state. Selected portions of the catheter are subject to the cold crystallization temperatures of the material in order to transform those portions into a more crystalline form.

US 2005/0277878 discloses a balloon catheter with a multilayered polymeric sleeve at a rapid exchange intermediate section. The polymeric sleeve has an outer layer formed of a polymer with a relatively low processing temperature and an inner layer.

US 2003/0139760 discloses a catheter assembly with an inflatable balloon on a portion of a shaft section. The balloon has tapered regions with increasing thickness. A fluid-tight bond is formed between the catheter shaft and the tapered regions, which are at least partially crystalline.

### Summary of the invention

Thus, an object of the present invention is to provide an improved implantable medical lead which alleviates the problem mentioned above.

Another object of the present invention is to provide an improved medical lead having a prolonged life time in comparison with prior art medical lead.

A further object of the present invention is to provide an improved method for selectively designing the properties of a material for use in a medical lead.

These and other objects are achieved by providing an implantable medical lead, a method for manufacturing thereof and use of an implantable polymer material or Elast-Eon 2A®-material in an implantable medical lead having the features defined in the independent claim. Preferred embodiments are defined in the dependent claims.

According to a first aspect of the present invention, there is provided an implantable medical lead for implantation in a patient comprising at least one electrical conductor connected to at least one electrode and/or sensor of said lead, said at least one conductor being arranged within a continuous sheet of a polymer material, wherein a distal portion of said lead is adapted to be located in or at a heart of said patient and wherein a proximal portion of said lead is connectable to an implantable medical device and arranged such that, when connected to said device, at least a part of said proximal portion is placed in close proximity to said medical device, wherein said lead is characterized in that: at least said proximal portion of said polymer sheet material is processed in at least a first heat process stage such that an inherent resistance to wear of said polymer sheet material is substantially maintained; and said distal portion of said polymer sheet material is processed in at least a second heat process stage in which a polymer morphology of said polymer material is altered such that an inherent flexibility of said polymer sheet material is substantially increased.

According to a second aspect of the present invention, there is provided a method for manufacturing of an implantable polymer sheet material for implantation in a patient, wherein a distal portion of said polymer sheet material is adapted to be located in or at a heart of said patient and wherein a proximal portion of said polymer sheet material is connectable to an implantable medical device and arranged such that, when connected to said device, at least a part of said proximal is placed in close proximity to said medical device, said method comprising the steps of: providing a continuous sheet of a polymer material; processing at least said proximal portion of said polymer sheet material in at least a first heat process stage such that an inherent resistance to wear of said polymer sheet material is substantially maintained; processing said distal portion in at least a second heat process stage in which a polymer morphology of said polymer material is altered such that an inherent flexibility of said polymer sheet material is substantially increased.

According to a third aspect of the present invention, there is provided a method for manufacturing of an implantable medical lead for implantation in a patient, wherein a distal portion of said lead is adapted to be located in or at a heart of said patient and wherein a proximal portion of said lead is connectable to an implantable medical device and arranged such that, when connected to said device, at least a part of said proximal is placed in close proximity to said medical device, said method comprising the steps of: providing at least one electrical conductor connected to at least one electrode and/or sensor of said lead; providing a continuous sheet of a polymer material; processing at least said proximal portion of said polymer sheet material in at least a first heat process stage such that an inherent resistance to wear of said polymer sheet material is substantially maintained; processing said distal portion in at least a second heat process stage in which a polymer morphology of said polymer material is altered such that an inherent flexibility of said polymer sheet material is substantially increased; and assembling said at least one conductor with said polymer sheet material.

According to a fourth aspect of the present invention, there is provided a use of an implantable polymer material having at least two portions with different morphology, wherein the polymer is a semi-crystalline copolymer having at least a soft amorphous segment and at least a hard crystalline segment being at least partially crystallized, in implantable medical lead according to the invention. In other words, use of such a material in anyone of the embodiments of the implantable medical lead.

According to a fifth aspect of the present invention, there is provided a use of an Elast-Eon 2A^{®}-material in an implantable medical lead according to the invention.

Thus, the present invention is based on the insight of using a single or continuous implantable polymer sheet material that can be heat processed to obtain selectable material properties at different parts of a processed material piece for achieving an implantable medical lead that is capable of uniting the contradicting requirements put upon such leads, i.e. a medical lead having a distal end portion which is flexible and a proximal portion sufficiently rigid to manoeuvre the distal end and wherein at least the proximal end portion is abrasion resistant. The implantable polymer sheet material functions is heat treated at individual portions to obtain an end portion having an high flexibility and wherein at least a proximal end portion is heat treated such that a high abrasion or wear resistance can be achieved. Thereby, an improved medical lead having one end for placement within or at a heart having a high degree of flexibility, and at least a portion of the opposite end, i.e. a proximal end, having a high resistance to wear or abrasion can be provided. Moreover, the use of a continuous sheet material and the fact that different parts of the sheet can be selectively processed eliminates unwanted joints or seems that could cause cracks or breakage. Thus, this enhances the reliability and durability even further.

The polymer sheet material used in the medical lead according to the first aspect enables an implantable medical lead having an enhanced abrasion or wear resistance at a selected portion, for example, the proximal end, which is in contact, or which at least frequently abuts, with medical device, and a flexible distal end which provides for a reliable and accurate connection between the lead and the heart. More specifically, a distal end, being secured to a portion of the heart tissue, which is flexible, enables a secure and reliable fixation point. Also, the flexible distal end portion facilitates an implantation or insertion of the lead.

In an embodiment of the first aspect of the invention, the polymer is a semi-crystalline copolymer having at least a soft amorphous segment and at least a hard crystalline segment being at least partially crystallized. It should be noted that the term "copolymer" as used herein is intended to refer to a polymer material that is derived from two (or more) monomers or monomeric species. Moreover, the term "semi-crystalline" as used herein is intended to refer to a polymer which is constituted by an amorphous and a crystalline region or section. A soft segment material is an elastomeric polymeric material that is amorphous and has a crystalline or glassy state that occurs at or above its intended use temperature, e.g. about 37 °C for implanted materials, and exhibits large degree of localized chain mobility. A hard segment is an elastomeric polymeric material that is crystalline or in an amorphous glassy state at and/or above the intended use temperature, and is characterized by a very low degree of localized chain mobility. The soft and hard regions or segments are phase separated meaning that the polymer material has elastomeric with elastomeric properties, such as elasticity, Thereby, the flexibility of the medical lead is enhanced which, in turn, facilitates and simplifies the insertion of the lead during the implantation procedure. Furthermore, during use, i.e. when the lead is implanted into a patient, the lead may easily follow the bodily movements.

In another embodiment of the first aspect of the invention, at least a portion of the amorphous segment comprises at least one flexible polymeric material from a group containing silicone, polyethers, polyethylene oxide, polyolefins, polycarbonates, or a combination thereof, and wherein at least a portion of the crystalline segment comprises at least one crystallizable polymeric material from a group containing aromatic urea, aromatic or aliphatic urethane. Such a material is often called a polyurethane material, a polyurea, or a polyurea-urethane. A preferred material comprises a linear block copolymer of hard and soft segments, and there are not chemical crosslinks between polymer chains to form a 3-D network, making these thermoplastics. At use temperature, crystallization between hard segments on the same or different chains give rise to a thermally-reversible network to give rise to the desired mechanical properties. Heating above the crystalline and glass transition temperatures gives rise to a melt which can be formed and processed as a thermoplastic according to known art. Hence, the preferred materials are classified as thermoplastic elastomers, of which thermoplastic urethanes is a preferred sub-group. A lead having such a polymer further enhances the elasticity of the polymer and thereby the flexibility of the lead.

In yet another embodiment of the first aspect of the invention, wherein the at least said proximal portion of said polymer sheet material is heat treated at a temperature interval from 50 to 100 °C during a period of about 30 minutes to about 5 hours and said distal portion of said polymer sheet material is heat treated at a temperature of at least 10 °C above the temperature of the first heat process stage during a period of at least about 5 minutes. Alternatively, said distal portion is heat treated at a temperature of about 120 °C during a period of about 30 minutes.

In yet another embodiment of the second or third aspect of the invention, the step of providing a continuous sheet of a polymer material comprises a step of providing a semi-crystalline copolymer having at least a soft amorphous segment and at least a hard crystalline segment being at least partially crystallized.

In another embodiment of the second or third aspect of the invention, the step of providing a continuous sheet of a polymer material comprises a step of providing a semi-crystalline copolymer having at least a soft amorphous segment where at least a portion thereof comprises at least one flexible polymeric material from a group containing silicone, polyethers, polyethylene oxide, polyolefins, polycarbonates, or a combination thereof, and having at least a hard crystalline segment where at least a portion thereof comprises at least one crystallizable polymeric material from a group containing aromatic urea, aromatic or aliphatic urethane.

Moreover, in another embodiment of the second or third aspect of the invention, the first heat process stage comprises heating within a temperature interval from about 50 to about 100 °C during a period of about 30 minutes to about 5 hours, and wherein the second heat process stage comprises heating at a temperature at least about 10 °C above the temperature of the first heat process stage during a period of at least about 5 minutes. Alternatively, the second heat process stage comprises heating at a temperature of about 120 °C during a period of about 30 minutes.

In yet another embodiment of the second or third aspect of the invention, the first and second heat process stage takes place simultaneously by means of a common oven that provides individual heat treatments to individual portions of the polymer sheet material. It is to be understood that such an oven may be arranged in various ways as long as a differentially heating is provided. For example, such an oven may be divided by oven walls such that the oven chamber is divided into at least to separate compartment, wherein each of these can provide individual heat treatments for the portion within the compartment.

The features that characterize the invention, both as to organization and to method of operation, together with further objects and advantages thereof, will be better understood from the following description used in conjunction with the accompanying drawings. It is to be expressly understood that the drawings is for the purpose of illustration and description and is not intended as a definition of the limits of the invention. These and other objects attained, and advantages offered, by the present invention will become more fully apparent as the description that now follows is read in conjunction with the accompanying drawings.

### Brief description of the drawings

Preferred embodiments of the invention will now be described in greater detail with reference to the accompanying drawings, in which
Fig. 1 illustrates the general principle of a medical lead in relation to a heart of a patient and a medical device;
Fig. 2 illustrates the relationship between abrasion resistance and hardness of a silicon elastomer material;
Fig. 3 illustrates the relationship between abrasion resistance and hardness of a polymer sheet material according to the invention; and
Fig. 4 illustrates the relationship between stiffness as a function of heat treatment temperature of a polymer sheet material according to the invention.
Fig. 5 shows a block diagram illustrating the principles of a process according to the present invention.

### Detailed description of exemplifying embodiments

The following is a description of exemplifying embodiments in accordance with the present invention. This description is intended for describing the general principles of the invention and is not to be taken in a limiting sense. Please note that like reference numerals indicate structures or elements having same or similar functions or constructional features.

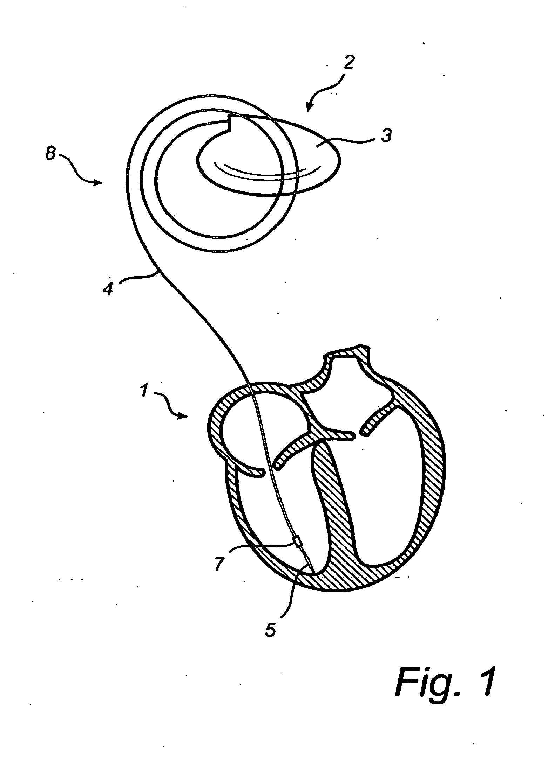

Referring first to Fig. 1, there is shown an implantable medical device or heart stimulator 2 in electrical communication with a human heart 1 via an implantable medical lead or cardiac lead 4 arranged for stimulation and sensing. Moreover, the heart stimulator 2 comprises electronic circuitry and a battery contained within a hermetically sealed pacemaker housing 3. The housing 3 comprises a metallic casing of a biocompatible material, for example, titanium, enclosing the electronic circuitry and battery, and a molded plastic header portion, comprising connector blocks and apertures for receiving the connectors at the proximal ends of the cardiac leads. Also, at the proximal end of the the lead, a coil of excess lead 8 is provided, which is to be implanted together with the medical device 2.

The electronic circuitry comprises at least one pulse generator for generating stimulation pulses, sensing circuitry for receiving cardiac signals sensed by the cardiac lead 2, and a controller. The controller controls both the sensing of cardiac signals and the delivery of stimulation pulses, for instance as to the duration, energy content and timing of the stimulation pulses.

The stimulation pulses generated by the pulse generator are transmitted via the cardiac lead 4 and delivered to the cardiac tissue by the use of tip electrodes positioned at the distal end 5 of the cardiac lead. Generally, the tip electrode acts as the cathode when the cardiac pulse is delivered. Furthermore, in unipolar cardiac systems, the casing 3 acts as the anode, while in bipolar cardiac systems, the anode is provided by an annular or ring electrode 7 arranged on the cardiac lead at a small distance from the tip electrode.

It should be noted that even though a ring electrode 7 is illustrated in the greatly simplified drawing of Fig. 1, the present invention is equally applicable to unipolar, bipolar, and multipolar systems. Thus, implantable leads with or without ring electrodes are equally contemplated without departing from the scope of the invention. Furthermore, even though only one lead 4 for attachment and stimulation in the right ventricle is illustrated in the drawing, the medical implant 2 may be connected to further leads, for instance for stimulation of the right atrium, the left atrium, and/or the left ventricle.

The implantable medical lead 4 according to the present invention preferably comprises at least one electrical conductor connected to at least one electrode and/or sensor of said lead, said at least one conductor being arranged within a continuous sheet of a polymer material. It should be noted that such a polymer sheet material may have the shape of a tube or the like provided with at least one lumen. In other words, the at least one electrical conductor is situated within a polymer sheet material, e.g. an isolating polymer tube

At least a portion of the proximal end of the lead has a maximized resistant to wear, or at least those parts which may be in contact with the device 2 when implanted. For example, that part of the lead that is implanted into the subcutaneous pocket is preferably subjected to a heat treatment such that the wear resistance of said polymer sheet material is substantially maintained. In Fig. 1, the lead 4 is provided with a wear or abrasion restistance surface property, which more or less equals a part of the lead 8 being located in the subcutanoues pocket and in proximity to the pocket, i.e. the proximal end portion. Preferably, the part of the lead that is less flexible, i.e. the proximal end, has a high resistant to wear. However, as is undersood, the wear resistant property may also be arranged in other ways. For example, only the part of the lead being placed within the pocket may be processed such the inherent wear resistance property is maintained. Similarly, the distal end or a distal portion of the lead is subjected to heat treatment such that the inherent flexibility property of the polymer sheet material is increased. In other words, the distal end, or at least a portion thereof, is more flexible than the proximal end, or at least a portion thereof. In Fig. 1, about 10 cm is flexible (not shown) of the about 50 cm long lead. The distal treatment should be applied to at least about 5 cm of the distal end, and preferably about 10 to about 25 cm, which is the distal portion of the lead that is situated within the heart.

### Experiment 1

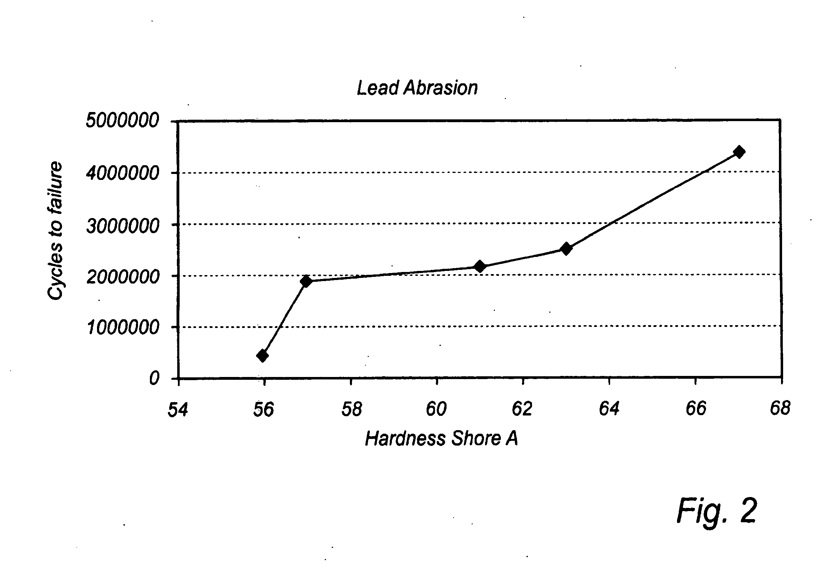

Fig. 2 presents experimental information showing that the hardness property and abrasion resistance property are related. The test results which relates to a silicone elastomer shows that within a group of otherwise chemically identical, the abrasion resistance increases with increasing hardness. The silicone elastomer or rubbers were cured and post-cured to achieve a specific hardness (Shore A). These were then tested in an abrasion test apparatus, which by St. Jude Medical internally is designated ES-1907 rev X1, designed for measuring the abrasion resistance of pacemaker lead bodies. For this type of abrasion, it has thus been demonstrated that harder materials, of otherwise identical composition as that of the present invention, have greater resistance to abrasion. Thus, it is beneficial to provide softness in the tip for flexibility, but retain hardness in the proximal end to optimize abrasion resistance.

### Experiment 2

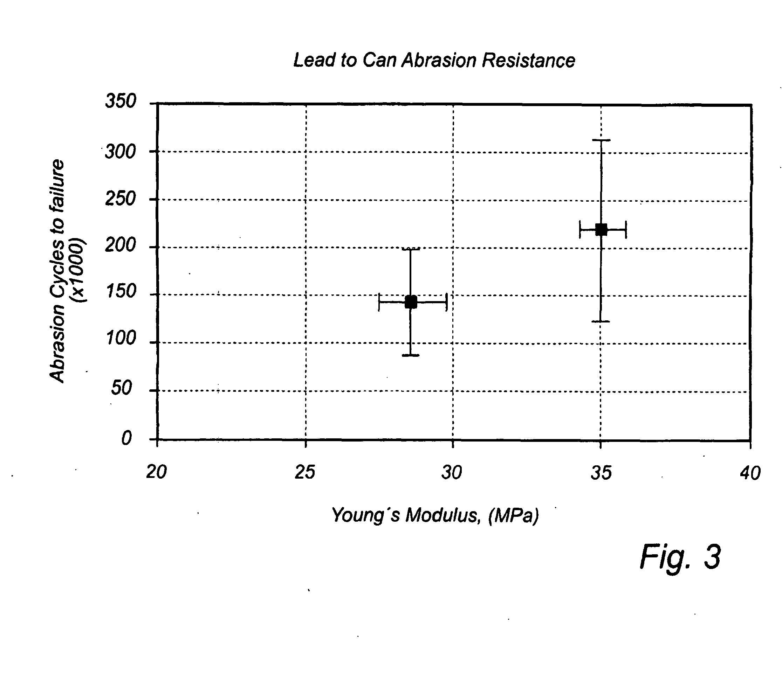

In Fig. 3, test results for a material according to an embodiment of the present invention, similar to that of the experiment 1, is shown. The graph in Fig. 3 presents abrasion resistance results on a pacemaker lead body made of an Elast-Eon material, more specifically an Elast-Eon 2A material. This material is provide by Aor-Tech. The purpose of such an experiment is to simulate the wear situation of a medical lead in abutment with a medical device can when implanted. The experiment was similiarly performed as experiment 1 in the way that the material was first subjected to heat treatment followed by an abrasion test. The abrasion test was performed according to a St. Jude Medical internal test method called 60010764 rev P02. The result shows that the lower hardness material, i.e. less stiff as indicated by lower Young's modulus, from a heat treatment at 120C/6hrs has a lower abrasion resistance compare to material treated at 85C/4h that has higher stiffness/hardness/modulus, and thus higher abrasion resistance.

According to an embodiment of the present invention, the at least proximal portion of the polymer sheet material has a hardness ranging from Shore 60A to Shore 80D. Thus, the at least proximal portion of the medical lead is provided with a inherent wear resistance property.

### Experiment 3

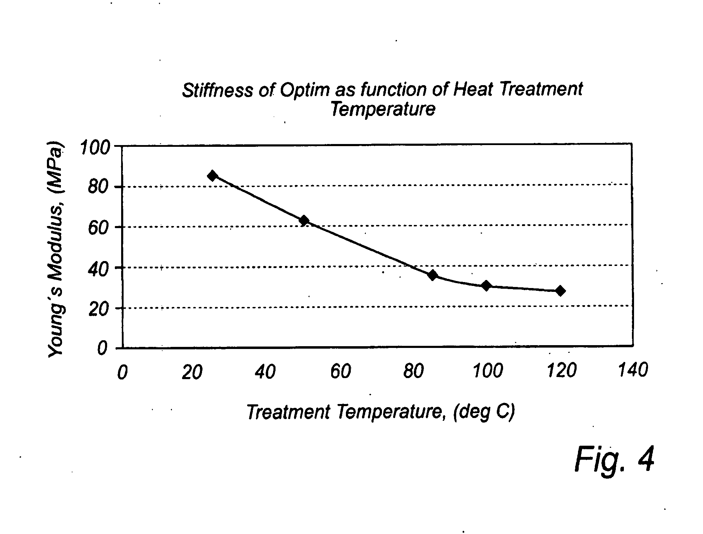

Fig. 4 shows the relationship between stiffness, indicated by Young's Modulus, and treatment temperature of a polymer sheet material according to an emobodiment of the invention. The experiment was performed by first heat treating an Elast-Eon 2A material in a conventional oven. In Fig. 4, the name Optim is used which is a name of the Elast-Eon 2A material used at St. Jude Medical. Thereafter, the stiffness was then measured by a conventional apparatus for measuring tensile properties of stress versus strain. Lloyd Instruments LRX plus ExT with 10N load cell tested on tubing in a mandril clamp with 100 mm gauge length. The graph shows that a higher treatment temperature results in a lower stiffness of the polymer material or Elast-Eon 2A provide by Aor-Tech.

### Heat treatment process

As is understood by the skilled person in the art, the heat treating process according to the present invention may be performed in number of alternative ways. In a example method for manufacturing of an implantable polymer sheet material which is to be implanted into a patient according to the present invention, first a continuous sheet of a polymer material is provided. Thereafter, at least a proximal portion of the polymer sheet material is processed in at least a first heat process stage. Thereby, an inherent resistance to wear of said polymer sheet material is substantially maintained. Thereafter, a distal portion in at least a second heat process stage is processed. The polymer morphology of this polymer material is altered such that an inherent flexibility of said polymer sheet material is substantially increased.

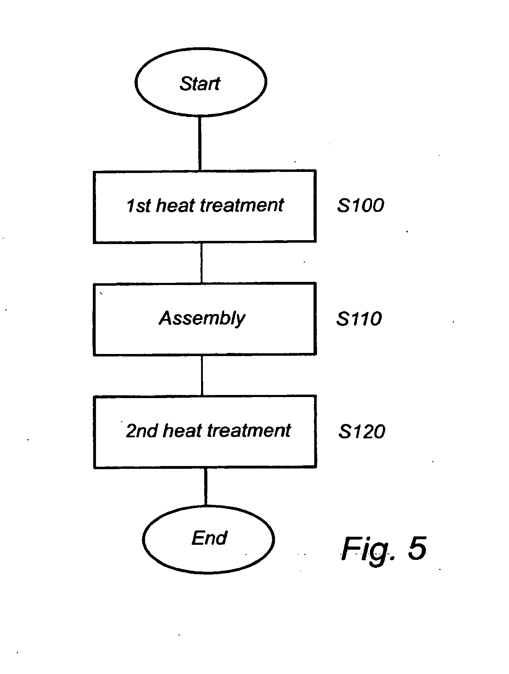

In Fig. 5, there is shown a schematic block diagram of a preferred process. First, at step S100, at least one polymer tube is placed in an oven having a temperature of about 85 °C. The at least one polymer tube is annealed in a batch process over the full length of the tube to stabilize its dimensions for 4 hours. The temperature and/or time parameter may be varied within the interval of the present invention, i.e. a temperature interval from about 50 °C to about 100 °C during a period of about 30 minutes to about 5 hours, to achieve a desired stabilizing effect of the dimensions. However, a preferred first process steps is, as mentioned above, to subject the tube to a first heat treatment step S100 at a temperature of about 85 °C for about 4 hours. Thereafter, at step S110, a lead is assembled using a processed polymer tube as an outer tube. This is not described in detail since is conventional practice within the art. After assembly of a lead, the lead is heat treated in a second heat treatment step S120. Preferably, a heating mantle or other suitable localized controlled temperature heat source is used to treat the sections of the lead, preferably about 10-25 cm of the distal lead end, where a higher degree of flexibility is desired. Temperatures selected influence the modulus or stiffness of the material in a controlled fashion. However, the second heat treatment is preferably performed at a temperature of about 120 °C for about 30 minutes.

As is understood, the tube or polymer sheet material may be gradually heated to attain a mechanical property gradient, i.e. the flexibility at the distal end is gradually decreased towards the proximal end, or at least up to that part of the lead that is not be wear or abrasion resistant.

Also, as is understood by the skilled artisan, the method may also comprise the step of providing at least one electrical conductor adapted to be connected to at least one electrode and/or sensor of the lead. Also, the step of assembling the medical lead may be done in a number of alternative ways. For example, the assembly of the lead may be performed after completion of the first and second heat treatment steps, or may be performed before the heat treatment. However, a preferred embodiment is to assemble the medical lead after the first heat treatment. During the first heat treatment stage, relaxation of internal stresses can occur which may alter the dimensions of the polymeric component sligthly. Thus, its useful to treat the entire tube prior to the assembly in order to control tolerances of components of the medical lead and device. Moreover, the assembling may also be performed in a number of alternative ways. For example, the conductors may be positioned within the polymer sheet material after the first heat treatment step, followed by the second heat treatment step. However, this assembling may or may not include mounting the sensor and/or electrode to the lead and also the medical device or control unit may also be mounted in the same assembly step.

Although an exemplary embodiment of the present invention has been shown and described, it will be apparent to those having ordinary skill in the art that a number of changes, modifications, or alterations to the inventions as described herein may be made. Thus, it is to be understood that the above description of the invention and the accompanying drawings is to be regarded as a non-limiting example thereof and that the scope of protection is defined by the appended patent claims.

## Claims

1. An implantable medical lead (4) for implantation in a patient comprising a continuous sheet of a polymer sheet material, wherein a distal portion (5) of said implantable medical lead (4) is adapted to be located in or at a heart (1) of said patient, comprising:
at least one electrical conductor connected to at least one electrode (7) and/or sensor of said implantable medical lead (4), said at least one electrical conductor being arranged within said continuous sheet, wherein a proximal portion of said implantable medical lead (4) is connectable to an implantable medical device (2) and arranged such that, when connected to said implantable medical device (2), at least a part of said proximal portion is placed in close proximity to said implantable medical device (2), **characterized in that**
at least a proximal portion of said polymer sheet material has been processed in at least a first heat process stage such that an inherent resistance to wear of said polymer sheet material is substantially maintained;
a distal portion (5) of said polymer sheet material has been processed in at least a second heat process stage in which a polymer morphology of said polymer sheet material is altered such that an inherent flexibility of said polymer sheet material is substantially increased; and
said polymer is a semi-crystalline copolymer having at least a soft amorphous segment and at least a hard crystalline segment being at least partially crystallized.

2. The implantable medical lead according to claim 1, wherein at least a portion of said soft amorphous segment comprises at least one flexible polymeric material from a group containing silicone, polyethers, polyethylene oxide, polyolefins, polycarbonates, or a combination thereof, and wherein at least a portion of said hard crystalline segment comprises at least one crystallizable polymeric material from a group containing aromatic urea, aromatic or aliphatic urethane.

3. The implantable medical lead according to any of preceding claims, wherein said at least a proximal portion of said polymer sheet material has been heat treated at a temperature interval from about 50 to about 100 °C during a period of about 30 minutes to about 5 hours and said distal portion (5) of said polymer sheet material has been heat treated at a temperature at least about 10 °C above the temperature of said first heat process stage during a period of at least about 5 minutes.

4. The implantable medical lead according to claim 3, wherein said distal portion (5) has been heat treated at a temperature of about 120 °C during a period of about 30 minutes.

5. A method for manufacturing of an implantable medical lead (4) for implantation in a patient, wherein a distal portion (5) of said implantable medical lead (4) is adapted to be located in or at a heart (1) of said patient, said method comprising the step of:
providing a continuous sheet of a polymer sheet material, wherein a proximal portion of said implantable medical lead (4) is connectable to an implantable medical device (2) and arranged such that, when connected to said implantable medical device (2), at least a part of said proximal portion is placed in close proximity to said implantable medical device (2), the step of providing said continuous sheet of said polymer sheet material comprises a step of:
providing a semi-crystalline copolymer having at least a soft amorphous segment and at least a hard crystalline segment being at least partially crystallized, said method comprising the steps of:
providing at least one electrical conductor connected to at least one electrode (7) and/or sensor of said lead (4), **characterized by**:
processing at least a proximal portion of said polymer sheet material in at least a first heat process stage such that an inherent resistance to wear of said polymer sheet material is substantially maintained;
processing a distal portion (5) of said polymer sheet material in at least a second heat process stage in which a polymer morphology of said polymer sheet material is altered such that an inherent flexibility of said polymer sheet material is substantially increased; and
assembling said at least one electrical conductor with said polymer sheet material.

6. The method according to claim 5, wherein the step of providing said continuous sheet of said polymer sheet material comprises a step of providing said semi-crystalline copolymer having said at least a soft amorphous segment where at least a portion thereof comprises at least one flexible polymeric material from a group containing silicone, polyethers, polyethylene oxide, polyolefins, polycarbonates, or a combination thereof, and having said at least a hard crystalline segment where at least a portion thereof comprises at least one crystallizable polymeric material from a group containing aromatic urea, aromatic or aliphatic urethane.

7. The method according to claim 5, wherein said first heat process stage comprises heating within a temperature interval from about 50 to about 100 °C during a period of about 30 minutes to about 5 hours, and wherein said second heat process stage comprises heating at a temperature at least 10 °C above the temperature of said first heat process stage during a period of at least 5 minutes.

8. The method according to any of claims 5 to 7, wherein said second heat process stage comprises heating at a temperature of about 120 °C during a period of about 30 minutes.

9. The method according to any of claims 5 to 8, wherein said first and second heat process stage takes place simultaneously by means of a common oven that provides individual heat treatments to individual portions of said polymer sheet material.

10. Use of an implantable polymer material having at least two portions with different morphology, wherein said polymer is a semi-crystalline copolymer having at least a soft amorphous segment and at least a hard crystalline segment being at least partially crystallized, in implantable medical lead (4) according to claim 1.

11. Use of an Elast-Eon 2A-material in an implantable medical lead (4) according to claim 1.

## Patentansprüche

1. Implantierbare medizinische Leitung (4) zur Implantation in einen Patienten, die eine durchgehende Lage aus einem Polymerfolienwerkstoff umfasst, wobei ein distaler Abschnitt (5) der implantierbaren medizinischen Leitung (4) so ausgelegt ist, dass er sich in oder an einem Herz (1) des Patienten befindet, umfassend:
mindestens einen elektrischen Leiter, der mit mindestens einer Elektrode (7) und/oder einem Sensor der implantierbaren medizinischen Leitung (4) verbunden ist, wobei der mindestens eine elektrische Leiter innerhalb der durchgehenden Lage angeordnet ist, wobei ein proximaler Abschnitt der implantierbaren medizinischen Leitung (4) an ein implantierbares Medizinprodukt (2) anschließbar ist und so angeordnet ist, dass, wenn er an das implantierbare Medizinprodukt (2) angeschlossen ist, zumindest ein Teil des proximalen Abschnitts sehr nah an dem implantierbaren Medizinprodukt (2) angeordnet ist, **dadurch gekennzeichnet, dass**
zumindest ein proximaler Abschnitt des Polymerfolienwerkstoffs in mindestens einer ersten Wärmeprozessstufe so behandelt wurde, dass ein natürlicher Verschleißwiderstand des Polymerfolienwerkstoffs im Wesentlichen aufrechterhalten wird;
ein distaler Abschnitt (5) des Polymerfolienwerkstoffs in mindestens einer zweiten Wärmeprozessstufe behandelt wurde, in der eine Polymermorphologie des Polymerfolienwerkstoffs verändert wird, sodass eine Eigenflexibilität des Polymerfolienwerkstoffs im Wesentlichen erhöht wird; und
das Polymer ein teilkristallines Copolymer mit mindestens einem weichen amorphen Segment und mindestens einem harten kristallinen Segment ist, das zumindest teilweise kristallisiert ist.

2. Implantierbares Medizinprodukt nach Anspruch 1, wobei mindestens ein Abschnitt des weichen amorphen Segments mindestens einen flexiblen Polymerwerkstoff aus einer Gruppe umfasst, die Silikon, Polyether, Polyethylenoxid, Polyolefine, Polycarbonate oder eine Kombination daraus enthält, und wobei mindestens ein Abschnitt des harten kristallinen Segments mindestens einen kristallisierbaren Polymerwerkstoff aus einer Gruppe umfasst, die aromatischen Harnstoff, aromatisches oder aliphatisches Urethan enthält.

3. Implantierbare medizinische Leitung nach einem der vorhergehenden Ansprüche, wobei der zumindest eine proximale Abschnitt des Polymerfolienwerkstoffs in einem Temperaturintervall von etwa 50 bis etwa 100 °C während eines Zeitraums von etwa 30 Minuten bis etwa 5 Stunden wärmebehandelt wurde und der distale Abschnitt (5) des Polymerfolienwerkstoffs bei einer Temperatur mindestens etwa 10 °C über der Temperatur der ersten Wärmeprozessstufe während eines Zeitraums von mindestens etwa 5 Minuten wärmebehandelt wurde.

4. Implantierbare medizinische Leitung nach Anspruch 3, wobei der distale Abschnitt (5) bei einer Temperatur von etwa 120 °C während eines Zeitraums von etwa 30 Minuten wärmebehandelt wurde.

5. Verfahren zur Herstellung einer implantierbaren medizinischen Leitung (4) zur Implantation in einen Patienten, wobei ein distaler Abschnitt (5) der implantierbaren medizinischen Leitung (4) so ausgelegt ist, dass er sich in oder an einem Herz (1) des Patienten befindet, wobei das Verfahren folgenden Schritt umfasst:
Bereitstellen einer durchgehenden Lage eines Polymerfolienwerkstoffs, wobei ein proximaler Abschnitt der implantierbaren medizinischen Leitung (4) an ein implantierbares Medizinprodukt (2) anschließbar ist und so angeordnet ist, dass, wenn er an das implantierbare Medizinprodukt (2) angeschlossen ist, zumindest ein Teil des proximalen Abschnitts sehr nah an dem implantierbaren Medizinprodukt (2) angeordnet ist, wobei der Schritt des Bereitstellens der durchgehenden Lage des Polymerfolienwerkstoffs folgenden Schritt umfasst:
Bereitstellen eines teilkristallinen Copolymers mit mindestens einem weichen amorphen Segment und mindestens einem harten kristallinen Segment, das zumindest teilweise kristallisiert ist,
wobei das Verfahren folgenden Schritt umfasst:
Bereitstellen von mindestens einem elektrischen Leiter, der mit mindestens einer Elektrode (7) und/oder einem Sensor der Leitung (4) verbunden ist; **gekennzeichnet durch**:
Behandeln zumindest eines proximalen Abschnitts des Polymerfolienwerkstoffs in mindestens einer ersten Wärmeprozessstufe, sodass ein natürlicher Verschleißwiderstand des Polymerfolienwerkstoffs im Wesentlichen aufrechterhalten wird;
Behandeln eines distalen Abschnitts (5) des Polymerfolienwerkstoffs in mindestens einer zweiten Wärmeprozessstufe, in der eine Polymermorphologie des Polymerfolienwerkstoffs verändert wird, sodass eine Eigenflexibilität des Polymerfolienwerkstoffs im Wesentlichen erhöht wird; und
Zusammensetzen des mindestens einen elektrischen Leiters und des Polymerfolienwerkstoffs.

6. Verfahren nach Anspruch 5, wobei der Schritt des Bereitstellens der durchgehenden Lage des Polymerfolienwerkstoffs einen Schritt des Bereitstellens des teilkristallinen Copolymers umfasst, das das mindestens eine weiche amorphe Segment aufweist, wo mindestens ein Abschnitt davon mindestens einen flexiblen Polymerwerkstoff aus einer Gruppe umfasst, die Silikon, Polyether, Polyethylenoxid, Polyolefine, Polycarbonate oder eine Kombination daraus enthält, und das mindestens eine harte kristalline Segment aufweist, wo mindestens ein Abschnitt davon mindestens einen kristallisierbaren Polymerwerkstoff aus einer Gruppe umfasst, die aromatischen Harnstoff, aromatisches oder aliphatisches Urethan enthält.

7. Verfahren nach Anspruch 5, wobei die erste Wärmeprozessstufe das Erwärmen innerhalb eines Temperaturintervalls von etwa 50 bis etwa 100 °C während eines Zeitraums von etwa 30 Minuten bis etwa 5 Stunden umfasst, und wobei die zweite Wärmeprozessstufe das Erwärmen bei einer Temperatur mindestens 10 °C über der Temperatur der ersten Wärmeprozessstufe während eines Zeitraums von mindestens 5 Minuten umfasst.

8. Verfahren nach einem der Ansprüche 5 bis 7, wobei die zweite Wärmeprozessstufe das Erwärmen bei einer Temperatur von etwa 120 °C während eines Zeitraums von etwa 30 Minuten umfasst.

9. Verfahren nach einem der Ansprüche 5 bis 8, wobei die erste und zweite Wärmeprozessstufe gleichzeitig mittels eines gemeinsamen Ofens stattfinden, der für die individuelle Wärmebehandlung einzelner Abschnitte des Polymerfolienwerkstoffs sorgt.

10. Verwendung eines implantierbaren Polymerwerkstoffs mit mindestens zwei Abschnitten mit unterschiedlicher Morphologie, wobei das Polymer ein teilkristallines Copolymer mit mindestens einem weichen amorphen Segment und mindestens einem harten kristallinen Segment ist, das zumindest teilweise kristallisiert ist, in der implantierbaren medizinischen Leitung (4) nach Anspruch 1.

11. Verwendung eines Elast-Eon 2A-Werkstoffs in einer implantierbaren medizinischen Leitung (4) nach Anspruch 1.

## Revendications

1. Fil médical implantable (4) destiné à l'implantation dans un patient, comprenant une feuille en continu en un matériau polymère en feuille, dans lequel une portion distale (5) dudit fil médical implantable (4) est adaptée pour être située dans ou sur un coeur (1) dudit patient, comprenant :
au moins un conducteur électrique connecté à au moins une électrode (7) et/ou un capteur dudit fil médical implantable (4), ledit au moins un conducteur électrique étant agencé dans ladite feuille en continu, une portion proximale dudit fil médical implantable (4) pouvant être connectée à un dispositif médical implantable (2) et agencée de telle sorte que, quand elle est connectée audit dispositif médical implantable (2), au moins une partie de ladite portion proximale est placée à proximité étroite dudit dispositif médical implantable (2), **caractérisé en ce que**
au moins une portion proximale dudit matériau polymère en feuille a été traitée dans au moins une première phase de processus thermique de sorte qu'une résistance à l'usure inhérente dudit matériau polymère en feuille est essentiellement maintenue ;
une portion distale (5) dudit matériau polymère en feuille a été traitée dans au moins une seconde phase de processus thermique dans laquelle une morphologie polymère dudit matériau polymère en feuille est modifiée de sorte qu'une flexibilité inhérente dudit matériau polymère en feuille est essentiellement augmentée ; et
ledit polymère est un copolymère semi-cristallin présentant au moins un segment amorphe mou et au moins un segment cristallin dur qui est au moins partiellement cristallisé.

2. Fil médical implantable selon la revendication 1, dans lequel au moins une portion dudit segment amorphe mou comprend au moins un matériau polymérique flexible d'un groupe comprenant de la silicone, des polyéthers, de l'oxyde de polyéthylène, des polyoléfines, des polycarbonates, ou une combinaison de ceux-ci, et dans lequel au moins une portion dudit segment cristallin dur comprend au moins un matériau polymérique cristallisable d'un groupe comprenant de l'urée aromatique, de l'uréthane aromatique ou aliphatique.

3. Fil médical implantable selon l'une quelconque des revendications précédentes, dans lequel ladite au moins une portion proximale dudit matériau polymère en feuille a été traitée thermiquement dans un intervalle de température d'environ 50 à environ 100 °C pendant une période d'environ 30 minutes à environ 5 heures et ladite portion distale (5) dudit matériau polymère en feuille a été traitée thermiquement à une température d'au moins environ 10 °C de plus que la température de ladite première phase de processus thermique pendant une période d'au moins environ 5 minutes.

4. Fil médical implantable selon la revendication 3, dans lequel ladite portion distale (5) a été traitée thermiquement à une température d'environ 120 °C pendant une période d'environ 30 minutes.

5. Procédé de fabrication d'un fil médical implantable (4) destiné à l'implantation dans un patient, dans lequel une portion distale (5) dudit fil médical implantable (4) est adaptée pour être située dans ou sur un coeur (1) dudit patient, ledit procédé comprenant l'étape consistant à :
mettre à disposition une feuille en continu en un matériau polymère en feuille, une portion proximale dudit fil médical implantable (4) pouvant être connectée à un dispositif médical implantable (2) et agencée de telle sorte que, quand elle est connectée audit dispositif médical implantable (2), au moins une partie de ladite portion proximale est placée à proximité étroite dudit dispositif médical implantable (2), l'étape consistant à mettre à disposition ladite feuille en continu dudit matériau polymère en feuille comprenant une étape consistant à :
mettre à disposition un copolymère semi-cristallin présentant au moins un segment amorphe mou et au moins un segment cristallin dur qui est au moins partiellement cristallisé, ledit procédé comprenant les étapes consistant à :
mettre à disposition au moins un conducteur électrique connecté à au moins une électrode (7) et/ou un capteur dudit fil (4), **caractérisé par** :
le traitement d'au moins une portion proximale dudit matériau polymère en feuille dans au moins une première phase de processus thermique de sorte qu'une résistance à l'usure inhérente dudit matériau polymère en feuille est essentiellement maintenue ;
le traitement d'une portion distale (5) dudit matériau polymère en feuille dans au moins une seconde phase de processus thermique dans laquelle une morphologie polymère dudit matériau polymère en feuille est modifiée de sorte qu'une flexibilité inhérente dudit matériau polymère en feuille est essentiellement augmentée ; et
l'assemblage dudit au moins un conducteur électrique avec ledit matériau polymère en feuille.

6. Procédé selon la revendication 5, dans lequel l'étape consistant à mettre à disposition ladite feuille en continu de matériau polymère en feuille comprend une étape consistant à mettre à disposition ledit copolymère semi-cristallin présentant ledit au moins un segment amorphe mou dont au moins une portion comprend au moins un matériau polymérique flexible d'un groupe comprenant de la silicone, des polyéthers, de l'oxyde de polyéthylène, des polyoléfines, des polycarbonates, ou une combinaison de ceux-ci, et présentant ledit au moins un segment cristallin dur dont au moins une portion comprend au moins un matériau polymérique cristallisable d'un groupe comprenant de l'urée aromatique, de l'uréthane aromatique ou aliphatique.

7. Procédé selon la revendication 5, dans lequel ladite première phase de processus thermique comprend le chauffage dans un intervalle de température d'environ 50 à environ 100 °C pendant une période d'environ 30 minutes à environ 5 heures, et dans lequel ladite seconde phase de processus thermique comprend le chauffage à une température d'au moins 10 °C de plus que la température de ladite première phase de processus thermique pendant une période d'au moins 5 minutes.

8. Procédé selon l'une quelconque des revendications 5 à 7, dans lequel ladite seconde phase de processus thermique comprend le chauffage à une température d'environ 120 °C pendant une période d'environ 30 minutes.

9. Procédé selon l'une quelconque des revendications 5 à 8, dans lequel lesdites première et seconde phases de processus thermique ont lieu simultanément au moyen d'un four commun qui effectue des traitements thermiques individuels sur des portions individuelles dudit matériau polymère en feuille.

10. Utilisation d'un matériau polymère implantable présentant au moins deux portions de morphologie différente, ledit polymère étant un copolymère semi-cristallin présentant au moins un segment amorphe mou et au moins un segment cristallin dur qui est au moins partiellement cristallisé, dans le fil médical implantable (4) selon la revendication 1.

11. Utilisation d'un matériau Elast-Eon 2A dans un fil médical implantable (4) selon la revendication 1.
